# EUROPEAN PATENT APPLICATION

(11) **EP 4 649 973 A1**
(43) Date of publication of application: **19.11.2025**
(21) Application number: 24176586.6
(22) Date of filing: 17.05.2024
(51) Int. Cl.: A61M 5/142

(54) **INFUSION DEVICE SYSTEM AND METHOD**

(71) Applicant: NXP B.V., 5656 AG Eindhoven (NL)
(72) Inventor: WIELAGE, Paul, 5656 AE Eindhoven (NL)
(74) Representative: Schmütz, Christian Klaus Johannes

(57) **Abstract**

The disclosure relates to an infusion device, system and method. Example embodiments include an infusion device (100) comprising: a substrate (101) having first and second opposing surfaces (102, 103), the first surface (102) having an adhesive layer (104) for attachment to skin of a user; an injector assembly (105) having a cannula (106) for delivery of liquid through an aperture (109) in the substrate (101); first and second electrodes (107a, 107b) on the first surface of the substrate on opposing sides of the aperture (109); and a controller (110) configured to apply a controllable electrostimulation voltage across the first and second electrodes (107a, 107b) following delivery of the liquid.

## Description

### Field

The disclosure relates to an infusion device, system and method.

### Background

With type 1 diabetes mellitus, the body is unable to produce insulin and is therefore unable to regulate blood sugar levels. Regular checks for blood sugar levels in combination with injections of insulin are therefore required to effectively manage blood sugar levels. Insulin may be administered using an insulin pump arranged to provide controllable amounts of insulin, which can be more convenient than conventional injections.

A problem with existing solutions for insulin delivery is that insulin is absorbed slowly following administration via subcutaneous injection. Insulin administered in this way may become active during a period of around 15 minutes to 4 hours after administration. As a result, maintaining blood sugar levels within defined limits can be difficult, even when using continuous monitoring of blood sugar levels. As a result, blood sugar levels can become too high (hyperglycaemia) or too low (hypoglycaemia) and can result in over-correction.

It is known that physical exercise can accelerate absorption of insulin, but this can be counter-productive because it also simulates digestion, resulting in a higher blood sugar level, as well as being inconvenient or not possible in many circumstances. Slow absorption can be compensated for by applying a higher dose, but this may result in hypoglycaemia.

### Summary

According to a first aspect there is provided an infusion device comprising:
a substrate having first and second opposing surfaces, the first surface having an adhesive layer for attachment to skin of a user;
an injector assembly having a cannula for delivery of a liquid through an aperture in the substrate;
first and second electrodes on the first surface of the substrate on opposing sides of the aperture; and
a controller configured to apply a controllable electrostimulation voltage across the first and second electrodes following delivery of the liquid.

The injector assembly may comprise a reservoir and a pump in fluid communication with the cannula for delivery of the liquid from the reservoir. The controller may be configured to control operation of the pump for delivery of the liquid.

The liquid may comprise a medication such as insulin, although the infusion device may alternatively be used for other medications.

The infusion device may comprise a battery connected to provide an electrical supply to the controller.

The infusion device may comprise four or more electrodes including the first and second electrodes, wherein the controller is configured to apply the controllable electrostimulation voltage across a selected pair of the electrodes.

According to a second aspect there is provided an infusion system comprising:
a blood sugar monitor;
an infusion device according to the first aspect; and
a delivery control device in communication with the blood sugar monitor and the infusion device,
wherein the delivery control device is configured to:
   receive a blood sugar reading from the blood sugar monitor;
   determine an amount of liquid to be delivered and a level of stimulation based on the blood sugar reading, wherein the liquid comprises insulin; and
   operate the infusion device to deliver the amount of liquid and the controllable electrostimulation voltage according to the required level of stimulation.

The injector assembly may comprise a reservoir and a pump in fluid communication with the cannula for delivery of the liquid from the reservoir, the delivery control device being configured to operate the controller to deliver the amount of liquid and controllable electrostimulation voltage.

The delivery control device may comprise a pump controller, a reservoir and a pump connectable to the cannula via a tube for delivery of liquid from the reservoir, the delivery control device being configured to operate the pump controller to deliver the amount of liquid to the cannula.

According to a third aspect there is provided a method of operating an infusion system comprising a blood sugar monitor, an infusion device according to the first aspect and a delivery control device in communication with the blood sugar monitor and the infusion device, the method comprising:
receiving by the delivery control device a blood sugar reading from the blood sugar monitor;
determining by the delivery control device an amount of liquid to be delivered and a level of stimulation based on the blood sugar reading, wherein the liquid comprises insulin; and
operating the delivery control device to deliver the amount of liquid and the controllable electrostimulation voltage according to the required level of stimulation.

The injector assembly may comprise a reservoir and a pump in fluid communication with the cannula for delivery of liquid from the reservoir, wherein the delivery control device operates the controller to deliver the amount of liquid and controllable electrostimulation voltage.

The delivery control device may comprise a pump controller, a reservoir and a pump in fluid communication with the cannula via a tube for delivery of liquid from the reservoir, wherein the delivery control device operates the pump controller to deliver the amount of liquid to the injector assembly.

According to a fourth aspect there is provided a computer program comprising instructions to cause a computer processor of a delivery control device to perform the method according to the third aspect.

There may be provided a computer program, which when run on a computer, causes the computer to configure any apparatus, including a circuit, controller, sensor, filter, or device disclosed herein or perform any method disclosed herein. The computer program may be a software implementation, and the computer may be considered as any appropriate hardware, including a digital signal processor, a microcontroller, and an implementation in read only memory (ROM), erasable programmable read only memory (EPROM) or electronically erasable programmable read only memory (EEPROM), as non-limiting examples. The software implementation may be an assembly program.

The computer program may be provided on a non-transitory computer readable medium, which may be a physical computer readable medium, such as a disc or a memory device, or may be embodied as a transient signal. Such a transient signal may be a network download, including an internet download.

These and other aspects of the invention will be apparent from, and elucidated with reference to, the embodiments described hereinafter.

### Brief description of Drawings

Embodiments will be described, by way of example only, with reference to the drawings, in which:
Figure 1 is a schematic sectional diagram illustrating an example infusion device;
Figure 2 is a schematic plan view of a skin-facing first surface of the example infusion device of Figure 1;
Figure 3 is a schematic diagram of an example infusion system;
Figure 4 is a flow diagram illustrating an example method of operating the infusion system of Figure 3;
Figure 5 is a schematic diagram of an example infusion device with a separate delivery device;
Figure 6 is a schematic plan view of an alternative example infusion device; and
Figure 7 is a schematic plan view of a further alternative example infusion device.

It should be noted that the Figures are diagrammatic and not drawn to scale. Relative dimensions and proportions of parts of these Figures have been shown exaggerated or reduced in size, for the sake of clarity and convenience in the drawings. The same reference signs are generally used to refer to corresponding or similar feature in modified and different embodiments.

### Detailed description of embodiments

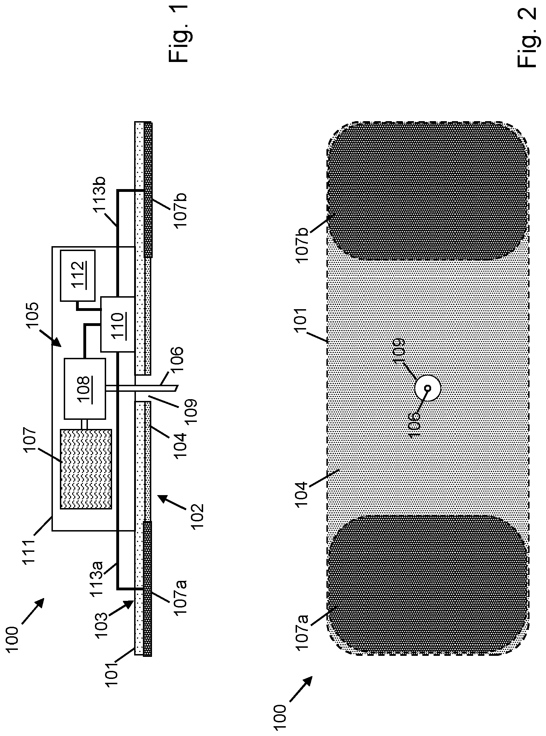

Figures 1 and 2 illustrate schematically an example infusion device 100. Figure 1 is a schematic sectional view of the device 100, while Figure 2 is a plan view of the skin-facing side of the device 100. The device 100 comprises a substrate 101 having first and second opposing surfaces 102, 103. A first skin-facing surface 102 has an adhesive layer 104 for attachment to the skin of a user. The substrate 101 has a central aperture 109 and first and second electrodes 107a, 107b on opposing sides of the aperture 109. The first and second electrodes 107a, 107b are on the skin facing side 102 of the substrate 101 to make electrical contact with the skin of the user when the device 100 is attached.

The infusion device 100 further comprises an injector assembly 105 having a delivery needle or cannula 106 that is in fluid communication with a reservoir 107. The cannula 106 is shown in Figure 1 passing through the aperture 109 in the substrate 101 to deliver subcutaneously to the user. In a practical implementation of the device 100, the cannula 106 may be provided in a retracted position and may be actuated to an injection position once the device 100 is attached. Various mechanisms for actuating the cannula 106 from a retracted position to an extended position are known.

In the example of Figure 1, a pump 108 in the device 100 is operable for delivery of a liquid comprising a medication from the reservoir 107 through the aperture 109 via the cannula 106 under control of the controller 110. Electrical power may be provided to the controller 110 by a battery 112, although in alternative implementations electrical power may be provided by a separate delivery control device, as described below. As well as controlling operation of the pump 108, the controller 110 may be configured to apply a controllable electrostimulation voltage across the first and second electrodes 107a, 107b following delivery of the medication to the user. This has the effect of providing electrostimulation of muscles in the area surrounding the aperture 109, which allows for faster absorption of the medication in a controlled manner following delivery. The electrostimulation may be activated only when required. Moderate electrostimulation may be applied during a short period of for example a few minutes following administration. A stronger stimulation may be applied in case of a rising measured blood sugar level. Electrostimulation may be suspended in case of a declining blood sugar level to prevent hypoglycaemia. Controlled stimulation can thereby assist with reducing the occurrence of lipohypertrophy, which is a common problem with diabetes therapy based on insulin infusion, because the insulin can be absorbed faster, particularly for larger doses.

The stimulation applied may for example comprise a train of pulses of a defined duration and amplitude, with a defined interval between the pulses. The train of pulses may for example start with a low amplitude that then increases. A trade-off is possible between using soft pulses for a longer amount of time and stronger pulses for a shorter amount of time.

Figure 1 illustrates the various components of the injector assembly 105 and the controller 110 within a housing 111 that is attached to the second surface 103 of the substrate 101. The substrate 101 extends outwards from either side of the housing 111 to provide a separation between the electrodes 107a, 107b so that electrostimulation can be applied to a larger area around the injection site corresponding to the location of the aperture 109. First and second electrical connections 113a, 113b connect the controller 110 to the first and second electrodes 107a, 107b respectively.

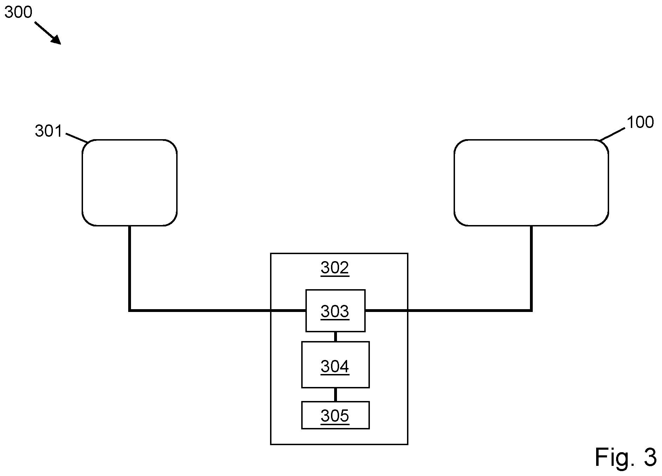

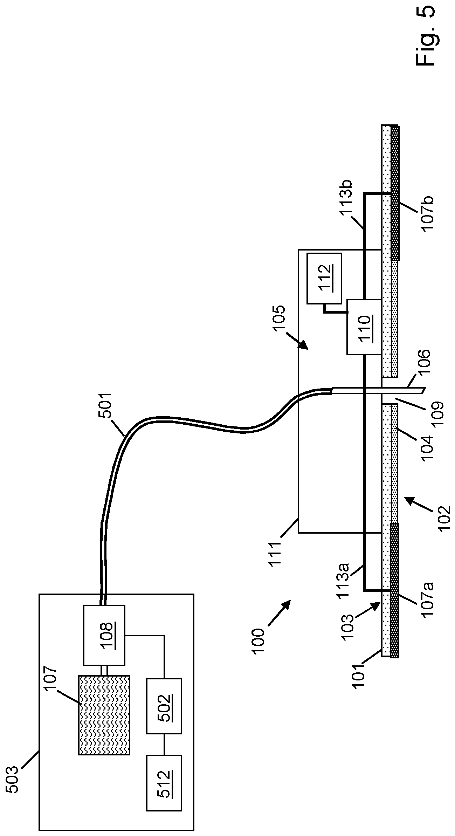

In alternative implementations, the reservoir 107 and pump 108 may be provided separately from the infusion device 100 and connected to the injector assembly 105 with a delivery tube 501, as illustrated in Figure 5. The controller 110 on the infusion device 100 may be configured to apply the electrostimulation voltage, while the pump 108 may be controlled to deliver liquid to the injector assembly 105 via the tube 501 by a separate pump controller 502. The pump 108, reservoir 107 and pump controller 502 may be provided as part of a delivery device 503, which may be, or be part of, the delivery control device 302 described below in relation to Figure 3. A battery 512 in the delivery device 503 provides electrical power to the pump controller 502. An advantage of separating the pump and reservoir from the infusion device 100 is that a longer lasting pump and a larger reservoir can be used, while the infusion device 100 can comprise disposable components and a smaller battery 112. Communication between the pump controller 502 in the delivery device 503 and the controller 110 in the infusion device 100 may be by wireless communication, for example via a Bluetooth connection. The controller 110 on the infusion device 100 can thereby operate to deliver an appropriate level of electrostimulation dependent on the time and quantity of liquid delivered by the pump 108.

Control of delivery of the medication may be provided by the controller 110 within the device 100 or external to the infusion device 100 and transmitted to the controller 110. In a practical implementation, a measure of blood sugar may be used in determining an amount of insulin to be delivered and an appropriate level of stimulation to be applied. Figure 3 illustrates schematically an infusion system 300 in which an infusion device 100 of the type described above is provided along with a blood sugar monitor 301 and a delivery control device 302. The blood sugar monitor 301 and infusion device 100 are both attached to the user's skin.

The delivery control device 302, which may for example be in the form of a handheld portable electronic device, is connected to the blood sugar monitor 301 and infusion device 100 via an input/output interface 303. Connections to the blood sugar monitor 301 and/or the infusion device 100 may be wired or wireless connections. A connection may for example be made via a wireless connection such as Bluetooth. In a practical implementation, a wireless connection may be made between the delivery control device 302 and the blood sugar monitor 301, since the blood sugar monitor 301 may be located separately from the delivery control device 302. A wired or wireless connection may be made between the delivery control device 302 and the infusion device 100 to enable the delivery control device 302 to control, and optionally provide power to, the infusion device 100. The delivery control device 302 may comprise a pump and reservoir, which is connected to the infusion device 100 with a tube, as shown in Figure 5.

A processor 304 in the delivery control device 302 receives the blood sugar reading from the blood sugar monitor 301 and determines an amount of insulin to be delivered, along with a level of stimulation to be applied based on the blood sugar reading. A memory 305 may be used to store data including blood sugar readings and other data and instructions for the processor 304 to make the appropriate determinations.

Once the processor 304 has determined the amount of insulin to be delivered and level of stimulation to be applied, the controller 110 on the infusion device 100 is operated by the delivery control device 302 to deliver the required amount of insulin and the controllable electrostimulation voltage to the electrodes 107a, 107b.

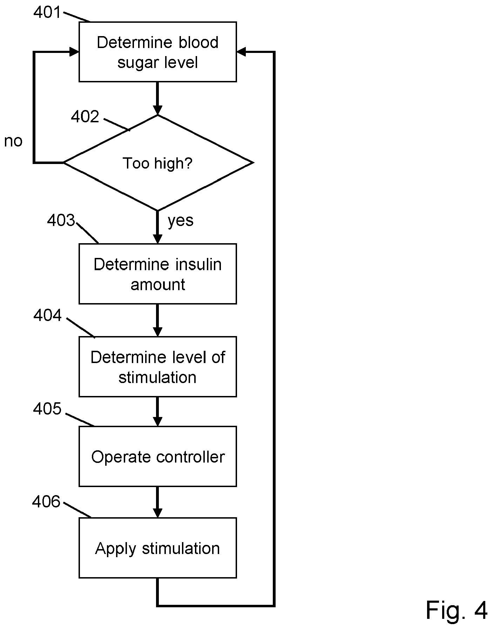

Figure 4 illustrates schematically a flow diagram illustrating a method of operation of the infusion system 300. In a first step 401, a blood sugar level is determined by the blood sugar monitor 301. The delivery control device 302 then determines whether the blood sugar level is too high at step 402. If not, the process then repeats by returning to step 401, for example after a predetermined interval. If, at step 402, the delivery control device 302 determines that the blood sugar level is too high, at step 403 a determination is made as to the amount of insulin to be delivered. The amount required may be determined according to a difference between a preset nominal blood sugar level and the measured level. At step 404 a determination is made regarding a level of electrostimulation to be applied, which may depend on the amount of insulin to be delivered and, if measured, the rate at which the blood sugar level is changing. As described above, if the blood sugar level is rising a level of stimulation may be higher than if the level is stable or falling. At step 405, the delivery control device 302 operates the controller 110 to deliver the required amount of insulin and at step 406 the corresponding required level of stimulation. In examples where the delivery control device 302 comprises the pump and reservoir, the delivery control device operates the pump directly to deliver insulin to the infusion device. Following this, the process repeats by returning to step 401, for example after a predetermined interval.

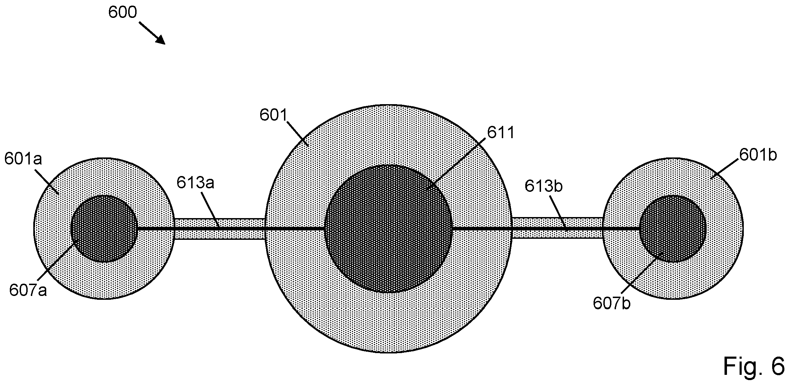

Figure 6 illustrates an alternative arrangement for an infusion device 600, in which the substrate 601 comprises first and second extension portions 601a, 601b. First and second electrodes 607a, 607b are disposed on the respective first and second substrate extension portions 601a, 601b. As with the example in Figure 1, the electrodes 607a, 607b are connected with respective electrical connections 613a, 613b. The extension portions 601a, 601b are arranged to be positioned apart from the housing 611 of the infusion set 600 so that their location can be selected to stimulate a specific set of muscles around the infusion site. The components of the infusion device 600 are otherwise similar to those in the other examples described above and illustrated in Figures 1 and 5.

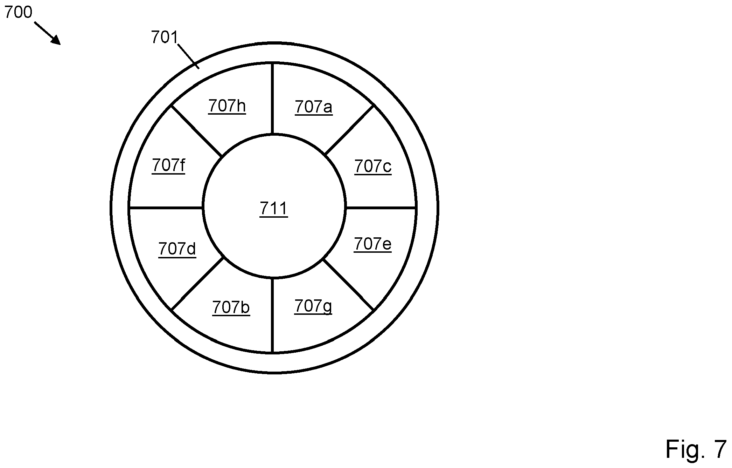

Figure 7 illustrates a further alternative arrangement for an infusion device 700, in which the substrate 701 comprises a plurality of pairs of electrodes 707a-g. The electrodes 707a-g may be in the form of a segmented ring as in Figure 7 or may be provided as separate electrode patches in a symmetrical arrangement around the housing 711. The housing 711 contains an infusion set of the type described above, with a controller that is in this case configured to apply an electrostimulation voltage between a selected pair of the electrodes 707a-g via respective electrical connections (not shown in Figure 7), for example between first and second electrodes 707a, 707b on opposing sides of the housing 711 and aperture. The controller may alternatively apply the electrostimulation voltage to selected groups of electrodes to provide a broader electrostimulation across the device 700, for example by applying the voltage between a first and second groups of electrodes, each group comprising one or more of the electrodes. The electrodes 707a-g can thereby be used to apply electrostimulation in multiple different ways and in different directions. The infusion set may comprise a pump and reservoir or may be connected to a delivery control device via a tube for delivery of liquid to the infusion set. In this example, 8 electrodes 707a-g are provided around the housing 711. In a general aspect, the infusion set may comprise four or more electrodes, and in a specific arrangement may comprise eight electrodes. A pair of the electrodes may be activated depending on the direction of electrostimulation required, which depends on the orientation of the muscles underneath the device 700 when in position. An advantage of this arrangement is that the orientation of the device 700 does not need to be aligned when being attached because the direction of electrostimulation can be selected after attachment. The direction of electrostimulation may for example be selectable by the user when the patch is in place, with the user selecting the pair of electrodes that provides the greatest effect, i.e. the orientation that stimulates the muscles most effectively.

An advantage of the various devices, systems and methods described herein is that electrostimulation can be applied selectively, and the absorption speed of the medication can be controlled as required by changing the duration and level of electrostimulation applied following administration. Improved control of blood sugar levels can thereby be more readily achieved in the case of insulin infusion.

From reading the present disclosure, other variations and modifications will be apparent to the skilled person. Such variations and modifications may involve equivalent and other features which are already known in the art of infusion systems, and which may be used instead of, or in addition to, features already described herein.

Although the appended claims are directed to particular combinations of features, it should be understood that the scope of the disclosure of the present invention also includes any novel feature or any novel combination of features disclosed herein either explicitly or implicitly or any generalisation thereof, whether or not it relates to the same invention as presently claimed in any claim and whether or not it mitigates any or all of the same technical problems as does the present invention.

Features which are described in the context of separate embodiments may also be provided in combination in a single embodiment. Conversely, various features which are, for brevity, described in the context of a single embodiment, may also be provided separately or in any suitable sub-combination. The applicant hereby gives notice that new claims may be formulated to such features and/or combinations of such features during the prosecution of the present application or of any further application derived therefrom.

For the sake of completeness it is also stated that the term "comprising" does not exclude other elements or steps, the term "a" or "an" does not exclude a plurality, a single processor or other unit may fulfil the functions of several means recited in the claims and reference signs in the claims shall not be construed as limiting the scope of the claims.

## Claims

1. An infusion device (100) comprising:
a substrate (101) having first and second opposing surfaces (102, 103), the first surface (102) having an adhesive layer (104) for attachment to skin of a user;
an injector assembly (105) having a cannula (106) for delivery of liquid through an aperture (109) in the substrate (101);
first and second electrodes (107a, 107b) on the first surface of the substrate on opposing sides of the aperture (109); and
a controller (110) configured to apply a controllable electrostimulation voltage across the first and second electrodes (107a, 107b) following delivery of the liquid.

2. The infusion device (100) of claim 1, wherein the injector assembly comprises a reservoir (107) and a pump (108) in fluid communication with the cannula (106) for delivery of liquid from the reservoir (107).

3. The infusion device (100) of claim 2, wherein the controller (110) is configured to control operation of the pump (108) for delivery of the liquid.

4. The infusion device (100) of any preceding claim, comprising a battery (112) connected to provide an electrical supply to the controller (110).

5. The infusion device (700) of any preceding claim, comprising four or more electrodes (707a-g) including the first and second electrodes, wherein the controller (110) is configured to apply the controllable electrostimulation voltage across a selected pair of the electrodes (707a-g).

6. An infusion system (300) comprising:
a blood sugar monitor (301);
an infusion device (100) according to claim 1; and
a delivery control device (302) in communication with the blood sugar monitor (301) and the infusion device (100),
wherein the delivery control device (302) is configured to:
receive a blood sugar reading from the blood sugar monitor (301);
determine an amount of liquid to be delivered and a level of stimulation based on the blood sugar reading, wherein the liquid comprises insulin; and
operate the infusion device (100) to deliver the amount of liquid and the controllable electrostimulation voltage according to the required level of stimulation.

7. The infusion system (300) of claim 6, wherein the injector assembly (105) comprises a reservoir (107) and a pump (108) in fluid communication with the cannula (106) for delivery of liquid from the reservoir (107), the delivery control device (302) being configured to operate the controller (110) to deliver the amount of liquid and controllable electrostimulation voltage.

8. The infusion system (300) of claim 6, wherein the delivery control device (302) comprises a pump controller (502), a reservoir (107) and a pump (108) connectable to the cannula (106) via a tube (501) for delivery of liquid from the reservoir (107), the delivery control device (302) being configured to operate the pump controller (502) to deliver the amount of liquid to the cannula (106).

9. A method of operating an infusion system (300) comprising a blood sugar monitor (301), an infusion device (100) according to claim 1 and a delivery control device (302) in communication with the blood sugar monitor (301) and the infusion device (100), the method comprising:
receiving by the delivery control device (302) a blood sugar reading from the blood sugar monitor (301);
determining by the delivery control device (302) an amount of liquid to be delivered and a level of stimulation based on the blood sugar reading, wherein the liquid comprises insulin; and
operating the delivery control device (302) to deliver the amount of liquid and the controllable electrostimulation voltage according to the required level of stimulation.

10. The method of claim 9, wherein the injector assembly (105) comprises a reservoir (107) and a pump (108) in fluid communication with the cannula (106) for delivery of liquid from the reservoir (107), wherein the delivery control device (302) operates the controller (110) to deliver the amount of liquid and controllable electrostimulation voltage.

11. The method of claim 9, wherein the delivery control device (302) comprises a pump controller (502), a reservoir (107) and a pump (108) in fluid communication with the cannula (106) via a tube (501) for delivery of liquid from the reservoir (107), wherein the delivery control device (302) operates the pump controller (502) to deliver the amount of liquid to the injector assembly (105).

12. A computer program comprising instructions to cause a processor (304) of a delivery control device (302) to perform the method according to any one of claims 9 to 11.
